# EUROPÄISCHE PATENTSCHRIFT

(11) **EP 3 180 051 B1**
(45) Veröffentlichungstag und Bekanntmachung des Hinweises auf die Patenterteilung: **10.07.2019**
(21) Anmeldenummer: 15756111.9
(22) Anmeldetag: 10.08.2015
(51) Int. Cl.: A61M 1/28, G16H 20/40

(54) **VORRICHTUNG ZUR BESTIMMUNG DES PERITONEALEN DRUCKES**
DEVICE FOR DETERMINING THE PERITONEAL PRESSURE
DISPOSITIF POUR DÉTERMINER LA PRESSION INTRAPÉRITONÉALE

(30) Priorität: 13.08.2014 DE 102014012024
(43) Veröffentlichungstag der Anmeldung: 21.06.2017
(73) Patentinhaber: Fresenius Medical Care Deutschland GmbH, 61352 Bad Homburg (DE)
(72) Erfinder: HOCHREIN, Torsten, 97478 Eschenau (DE); HEDMANN, Frank, 97332 Volkach (DE)
(74) Vertreter: Herrmann, Uwe
(86) Internationale Anmeldenummer: PCT/EP2015/001647
(87) Internationale Veröffentlichungsnummer: WO 2016/023632

(56) Entgegenhaltungen:
- DE-A1-102008 031 662
- JP-A- 2000 084 070
- US-B1- 6 228 047

## Beschreibung

Die vorliegende Erfindung betrifft eine Vorrichtung zur Bestimmung des peritonealen Druckes im Bauchraum eines Patienten und/oder zur Bestimmung des Entleerungszustandes des Bauchraums eines Patienten, wobei die Vorrichtung zumindest eine Pumpe zur Förderung einer Dialyselösung in den Bauchraum sowie zumindest eine Messeinrichtung zum Messen des Druckes in der Dialyselösung umfasst.

Im Rahmen einer Peritoneal-Dialysebehandlung wird ein Volumen an Dialyseflüssigkeit in den Bauchraum eines Patienten eingeführt und dort für eine bestimmte Verweildauer belassen. Das Peritoneum wirkt als semipermeable Membran, die einen Stoffübergang von dem Blut in die Dialyseflüssigkeit ermöglicht, wodurch eine Blutreinigung erreicht wird.

Während des vollständigen Auslaufes, d.h. während des Abführens der Dialyselösung aus dem Bauraum entsteht ein Unterdruck. Dieser in der Dialyselösung gemessene Druck setzt sich aus dem dynamischen Druck bedingt durch die Strömung der Dialyselösung, aus dem statischen Druck bedingt durch die Lage des Patienten sowie aus dem Druck im Bauchraum, d.h. aus dem peritonealen Druck zusammen.

Aus der DE 10 2008 031 662 A1 ist eine Vorrichtung zur Peritonealdialyse mit einer Einrichtung zur regelmäßigen Abgabe und Wiederaufnahme von Dialysat bekannt, wobei die Vorrichtung eine Druckmeßeinrichtung zur Messung des intraperitonealen Drucks aufweist.

Da der gemessene Druck somit aus mehreren Komponenten besteht, kann das Dialysegerät keine eindeutige Zuordnung treffen, d.h. keine Aussage darüber treffen, wie hoch der peritoneale Druck ist, der ein Maß für die Entleerung des Bauchraums des Patienten ist. Auch lässt sich aus dem gemessenen Gesamtdruck nicht ohne weiteres eine Aussage dazu treffen, inwieweit der Bauchraum entleert ist.

Der vorliegenden Erfindung liegt somit die Aufgabe zugrunde, eine Vorrichtung der eingangs genannten Art dahingehend weiterzubilden, dass eine Bestimmung des peritoneales Druckes und/oder des Entleerungszustandes des Patienten möglich ist.

Diese Aufgabe wird durch eine Vorrichtung mit den Merkmalen des Anspruchs 1 gelöst. Danach ist vorgesehen, dass die Vorrichtung wenigstens eine Steuereinheit umfasst, die derart ausgebildet ist, dass sie die Pumpe sowie die Messeinrichtung derart ansteuert, dass zum Erhalten eines ersten Druckmesswertes eine erste Messung des Druckes der Dialyselösung durch die Messeinrichtung bei stehender Pumpe vorgenommen wird, dass sie im Anschluss daran die Pumpe zur Förderung eines Teilvolumens des Gesamteinlaufvolumens der Dialyselösung in den Bauchraum in Betrieb setzt und die Pumpe dann anhält. Die Steuereinheit ist des Weiteren derart ausgeführt, dass zum Erhalten eines zweiten Druckmesswertes eine zweite Messung des Druckes der Dialyselösung durch die Messeinrichtung vorgenommen wird.

Die Steuereinheit ist somit derart ausgebildet, dass sie ein bestimmtes Verfahren durchführt, wobei dieses Verfahren eine erste Druckmessung in der Dialyselösung umfasst, wenn die Dialyselösung nicht strömt, wobei das Verfahren des Weiteren das Einlaufen eines Teilvolumens des für den Patienten bestimmten Gesamteinlaufvolumens mittels einer Pumpe, das Anhalten der Pumpe und eine zweite Druckmessung in der Dialyselösung umfasst.

Ist der Differenzdruck zwischen den beiden Druckmessungen gering, herrschte kein oder kein wesentlicher Unterdruck im Bauchraum, d.h. der gemessene Druck ist auf die Lage des Patienten zurückzuführen.

Ist der Differenzdruck zwischen den beiden Druckmessungen groß, herrschte ein Unterdruck im Bauchraum und der gemessene erste Druckwert gibt den peritonealen Druck wieder.

Der Förderdruck einer Pumpe, d.h. der dynamische Druck spielt bei den beiden Druckmessungen keine Rolle, da die Dialyselösung aufgrund der stehenden Pumpe ruht.

Durch die beschriebene Vorgehensweise ist es möglich, eine Bestimmung des peritonealen Drucks vorzunehmen, der dann beispielsweise in der Vorrichtung gespeichert werden kann oder in einer Auswerteeinrichtung verarbeitet werden kann.

Die beschriebene Vorgehensweise lässt des Weiteren Rückschlüsse auf den Entleerungszustand des Bauchraums zu. Ist der Differenzdruck zwischen den beiden Druckmessungen groß, ist dies ein Indikator dafür, dass der Bauchraum bei der vorausgegangen Entleerung vollständig entleert wurde. Ist der Differenzdruck zwischen den beiden Druckmessungen jedoch gering, zeigt dies, dass noch ein bestimmtes Volumen der Dialyselösung im Bauchraum vorhanden war bzw. ist.

Durch die erfindungsgemäße Vorrichtung ist es möglich, den im Verlauf einer Behandlung gemessenen Druck zuzuordnen. Wie ausgeführt kann anhand der beiden Druckmessungen festgestellt werden, ob der gemessene Druck der peritoneale Druck ist und/oder welchen Entleerungszustand der Bauchraum aufweist.

Das durch die Vorrichtung in den Bauchraum geförderte Volumen kann in dem Bauchraum verbleiben. Nach der erfolgten zweiten Druckmessung kann der verbleibende Teil des Gesamteinlaufvolumens in den Bauchraum gefördert werden, wozu die Pumpe durch die genannte Steuereinheit entsprechend angesteuert wird.

Das geförderte Teilvolumen kann größer gewählt werden, so dass ein "lokaler Druck" in einer "Peritoneum-Tasche" ausgeschlossen werden kann.

Ein weiterer Vorteil besteht darin, dass auftretende Druckalarme nicht mehr durch einen Nutzer der Vorrichtung quittiert werden müssen, was die Anzahl der Interaktionen zwischen Vorrichtung und dem Nutzer verringert. Dies ist insbesondere bei der nächtlichen Durchführung einer mit der Vorrichtung durchgeführten Peritoneal-Dialyse wünschenswert.

Das eigenständige Zurücksetzen von Fehlern wird dadurch ermöglicht, dass die Vorrichtung durch die zweite Druckmessung bzw. durch den Vergleich der beiden Druckmessungen die Fähigkeit besitzt, den gemessenen Druckwert einschätzen zu können. Dadurch lässt sich die Anzahl der Druckalarme reduzieren bzw. auftretende Druckalarme können durch die Vorrichtung selbst einer Kontrolle auf Plausibilität unterzogen werden und ggf. durch die Vorrichtung selbst quittiert werden.

Wäre eine solche Plausibilitätskontrolle nicht möglich und würde die Vorrichtung einen Alarm ohne eine solche Kontrolle zurücksetzen, birgt dies das Risiko, dass gefährdende Situationen dem Nutzer nicht signalisiert werden bzw. diese Situationen nicht aufgelöst oder sogar verstärkt werden.

Grundsätzlich können genau zwei Druckmesswerte oder auch mehr als zwei Druckmesswerte ermittelt werden. Im zweiten Fall können somit auch mehr als zwei Druckmesswerte miteinander verglichen werden, um Rückschlüsse auf den Entleerungszustand des Bauchraums und/oder auf den peritonealen Druck ziehen zu können.

Die Steuereinheit der Vorrichtung kann derart ausgebildet sein, dass das durch die Pumpe geförderte Teilvolumen einen bestimmten Prozentsatz des gesamten, dem Patienten zu verabreichenden Gesamteinlaufvolumens nicht überschreitet oder genau einem solchen Prozentsatz entspricht. Denkbar ist es beispielsweise, dass das Teilvolumen, das zwischen den beiden Druckmessungen in den Bauchraum eingeführt wird, 5 % des für den Patienten bestimmten Gesamteinlaufvolumens beträgt.

Auch ist es denkbar, dass die Steuereinheit derart ausgebildet ist, dass das durch die Pumpe geförderte Teilvolumen einem bestimmten Absolutwert entspricht oder diesen nicht überschreitet. So kann beispielsweise vorgegeben werden, dass das genannte Teilvolumen z.B. 100 ml beträgt.

Erfindungsgemäß umfasst die Vorrichtung zumindest eine Auswerteeinheit, die derart ausgebildet ist, dass sie die Differenz zwischen beiden Druckmesswerten ermittelt und aus der ermittelten Differenz den Entleerungszustand des Bauchraums bestimmt. Wie oben ausgeführt, kann die Auswerteeinheit derart ausgebildet sein, dass sie auf einen entleerten Bauchraum schließt, wenn die Druckdifferenz groß ist. Dies kann beispielsweise optisch und/oder akustisch oder auf sonstige Weise an der Vorrichtung signalisiert werden.

Die Auswerteeinheit ist derart ausgebildet, dass auf einen entleerten Bauchraum geschlossen wird, wenn die Differenz zwischen den beiden Druckwerten einen bestimmten absoluten oder relativen Grenzwert übersteigt. Beträgt der Betrag die Differenz beispielsweise 50 mbar oder mehr, wird darauf geschlossen, dass der Bauchraum bei der ersten Druckmessung entleert war.

Anstatt einen absoluten Druckwert zugrunde zu legen, kann auch vorgesehen sein, dass die Druckdifferenz in Relation zu dem gemessenen ersten oder zweiten Druckwert gesetzt wird und die Relation dann zur Bestimmung des Entleerungszustandes herangezogen wird.

Die vorgenannte Auswerteeinheit oder auch eine weitere Auswerteeinheit ist derart ausgebildet, dass sie die Differenz zwischen beiden Druckwerten ermittelt und aus der ermittelten Differenz den peritonealen Druck bestimmt. Ist die Differenz groß, kann festgestellt werden, dass der erste gemessene Druckwert den peritonealen Druck darstellt. Ob die Differenz groß ist, kann beispielsweise dadurch bestimmt werden, dass geprüft wird, ob die Differenz zwischen den beiden Druckwerten einen bestimmten absoluten oder relativen Grenzwert übersteigt.

Weiterhin kann die Vorrichtung wenigstens einen Speicher aufweisen, in dem der peritoneale Druck gespeichert wird. Dieser gespeicherte Wert kann für die folgenden Entleerungs- und Füllzyklen zugrunde gelegt werden, so dass die Vorrichtung gemessene Druckwerte stets dahingehend überprüfen kann, ob es sich um den peritonealen Druck im Bauchraum handelt, was zur Folge hat, dass die Pumpe durch die Steuereinheit bei Erreichen dieses Druckwertes gestoppt wird, da der Bauchraum entleert ist.

Die vorliegende Erfindung betrifft des Weiteren ein Peritonealdialysegerät, d.h. ein Dialysegerät mit Mitteln zur Durchführung einer Peritonealdialysebehandlung umfassend wenigstens eine Vorrichtung gemäß einem der Ansprüche 1 bis 5. Die Pumpe und/oder die Messeinrichtung können die Pumpe sowie die Messeinrichtung des Peritonealdialysegerätes sein, d.h. die Vorrichtung kann einen integralen Bestandteil des Peritonealdialysegerätes bilden. Grundsätzlich ist jedoch auch denkbar, dass es sich bei der Pumpe und/oder bei der Messeinrichtung der Vorrichtung um andere Elemente handelt als bei der Pumpe und/oder bei der Messeinrichtung des Peritonealdialysegerätes.

Das Peritonealdialysegerät kann Mittel zur Erzeugung eines Alarms in Abhängigkeit des gemessenen Druckes aufweisen, wobei die Mittel derart ausgebildet sind, dass der ermittelte peritoneale Druck bei der Erzeugung des Alarms berücksichtigt wird.

Die vorliegende Offenbarung betrifft des Weiteren ein Verfahren, nicht Teil des Anspruchgegenstandes, zur Bestimmung des peritonealen Druckes im Bauchraum eines Patienten und/oder zur Bestimmung des Entleerungszustandes des Bauchraums eines Patienten, wobei das Verfahren das Einführen von Dialyselösung in den Bauchraum des Patienten umfasst und wobei das Verfahren die folgenden Schritte aufweist:
a. Messung eines ersten Druckwertes der Dialyselösung, während die Pumpe zur Förderung der Dialyselösung steht,
b. Förderung eines Teilvolumens des Gesamteinlaufvolumens der Dialyselösung in den Bauchraum,
c. Anhalten der Pumpe und Messung eines zweiten Druckwertes bei stehender Pumpe.

Die Vorrichtung bzw. das Peritonealdialysegerät gemäß der Erfindung weist vorzugsweise Mittel auf, die geeignet und bestimmt sind, das Verfahren auszuführen.

Wie oben ausgeführt, kann vorgesehen sein, dass das durch die Pumpe geförderte Teilvolumen einen bestimmten Prozentsatz des gesamten, dem Patienten zu verabreichenden Gesamteinlaufvolumens nicht überschreitet oder genau einem solchen Prozentsatz entspricht. Das durch die Pumpe geförderte Teilvolumen kann einem bestimmten Absolutwert entsprechen oder es kann vorgesehen sein, dass dieser nicht überschritten wird.

Das Verfahren, nicht Teil des Anspruchgegenstandes, kann des Weiteren derart ausgestaltet sein, dass die Differenz zwischen beiden Druckmesswerten ermittelt wird und aus der ermittelten Differenz auf den Entleerungszustand des Bauchraums geschlossen wird. Dabei kann auf einen entleerten Bauchraum geschlossen werden, wenn die Differenz zwischen den beiden Druckwerten einen bestimmten absoluten oder relativen Grenzwert übersteigt.

Auch ist es denkbar, dass die Differenz zwischen beiden Druckwerten ermittelt wird und aus der ermittelten Differenz der peritoneale Druck bestimmt wird. Dieser entspricht dem ersten Messwert, vorausgesetzt, dass die Differenz zwischen den beiden Druckwerten groß ist, d.h. einen bestimmten absoluten oder relativen Grenzwert übersteigt.

Vorzugsweise ist vorgesehen, dass der peritoneale Druck dem gemessenen ersten Druckwert gleichgesetzt wird, wenn die Differenz zwischen den beiden Druckwerten einen bestimmten absoluten oder relativen Grenzwert übersteigt.

Der auf diese Weise ermittelte peritoneale Druck kann gespeichert werden. Er kann im weiteren Verlauf der Behandlung zur Einordnung der gemessenen Druckwerte und zur Feststellung herangezogen werden, ob der Bauchraum entleert ist oder ob dies nicht der Fall ist.

Weitere Einzelheiten und Vorteile der Erfindung werden anhand eines in der Zeichnung dargestellten Ausführungsbeispiels näher erläutert.

Es zeigen:
- Figur 1:: den zeitlichen Soll- und Ist-Verlauf des in dem Bauchraum befindlichen Füllvolumens während einer Peritonealdialysebehandlung und
- Figur 2:: die zu den Messpunkten in Figur 1 gehörenden Druckwerte.

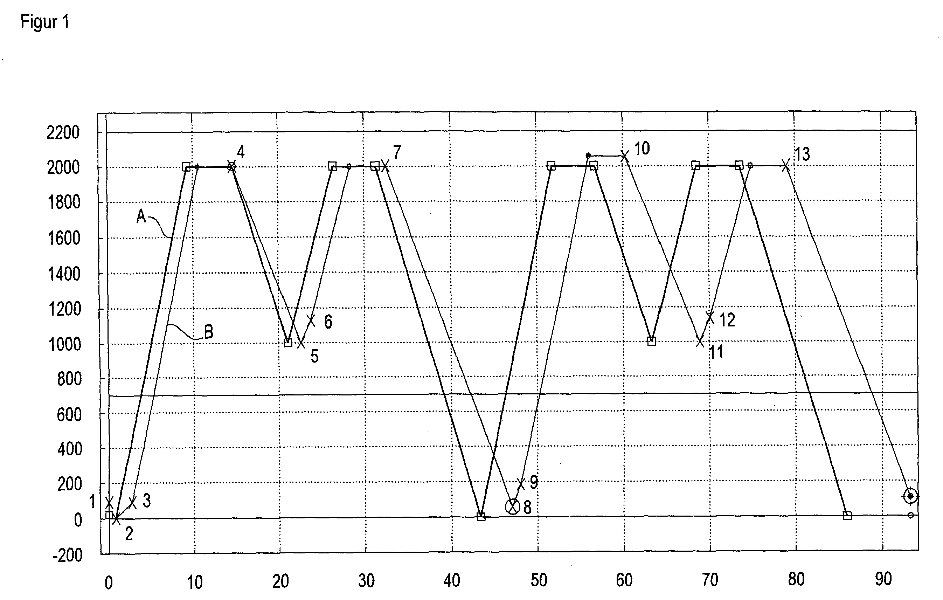

Figur 1 zeigt das Behandlungsvolumen, d.h. das dem Patienten verabreichte Volumen der Dialyselösung im Rahmen einer Peritonealdialysebehandlung über die Zeit.

Dabei stellt die Linie A den zeitlichen Verlauf des vorgeschriebenen Behandlungsvolumens und die Linie B den zeitlichen Verlauf des tatsächlich geförderten Volumens, d.h. im Bauchraum des Patienten befindliche Volumen der Dialyselösung dar.

Figur 1 zeigt eine sogenannte Tidal-Behandlung, die in zwei Basis und in zwei Tidal-Zyklen aufgeteilt ist. Bei einem Basiszyklus wird der Bauchraum des Patienten komplett von der Dialyselösung entleert, bei den Tidal-Zyklen verbleibt stets ein Restvolumen der Dialyselösung im Bauchraum, d.h. es findet keine vollständige Entleerung des Bauchraums von Dialyselösung statt.

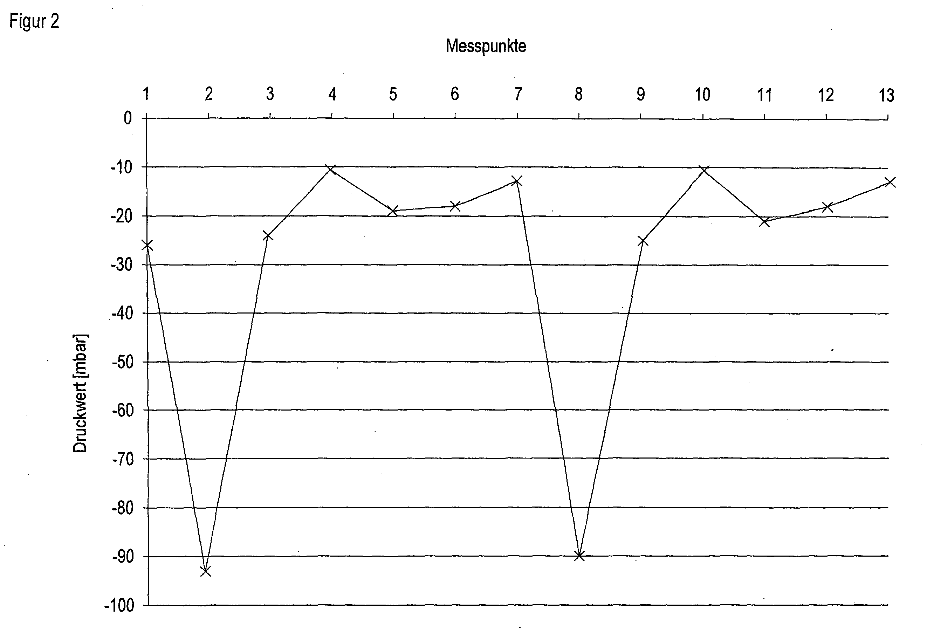

Die Punkte X1 bis X13 auf der Linie B markieren die Zeitpunkte der Druckmessungen. Die dabei erhaltenen Druckwerte ergeben sich aus Figur 2.

Bei dem Messpunkt X2 ist der Bauchraum vollständig entleert, d.h. es befindet sich keine Dialyselösung mehr im Bauchraum. Der Druck nimmt wie aus Figur 2 ersichtlich einen Minimalwert an. In dem hier gezeigten Beispiel beträgt der Unterdruck ca. 92 mbar. Während der Entleerung nimmt der Druck im Bauchraum bzw. in der Dialyselösung stark ab, wie dies aus einem Vergleich der Messwerte X1 und X2 hervorgeht.

Wird ausgehend von dem entleerten Zustand des Bauchraums, d.h. ausgehend von Punkt X2 ein Teilvolumen von Dialyselösung in den Bauchraum eingeführt, ergibt sich der Punkt X3. In dem hier gezeigten Beispiel beträgt das Teilvolumen ca. 100 ml.

Der Druckanstieg zwischen den Punkten X2 und X3 ist erheblich, wie sich dies aus Figur 2 entnehmen lässt. Dieselbe Situation ergibt sich für den vollständig entleerten Zustand bei Messpunkt X8. Auch hier führt die Zufuhr einer im Vergleich zum Gesamteinlaufvolumen (ca. 2 I) geringen Menge von Dialyselösung (ca. 100 ml) zu einem erheblich Anstieg des gemessenen Druckes. Diese große Druckdifferenz lässt den Schluss zu, dass eine komplette Entleerung des Bauchraums erfolgte. Auf diese Weise kann erkannt werden, dass der Bauchraum entleert wurde. Abgesehen davon kann der peritoneale Druck festgestellt werden, dass d.h. der Druck im entleerten Zustand des Bauchraums (in dem Ausführungsbeispiel ca. 93 mbar Unterdruck an den Messpunkten X2 und X8).

Die Druckmessung bei X2 und X8 erfolgt bei stehender Pumpe. Anschließend wird ein Teilvolumen der Dialyselösung eingeführt, die Pumpe angehalten und erneut der Druck gemessen (Druckmessung bei X3 und bei X9). Aus der in Figur 2 dargestellten Druckdifferenz kann wie ausgeführt rückgeschlossen werden, dass der Bauchraum entleert war.

Liegt hingegen noch ein Restvolumen im Bauchraum vor, wie dies bei den Messpunkten X5 und X11 der Fall ist und wird das Verfahren dann ebenfalls durchgeführt, d.h. Druckmessung in der stehenden Dialyselösung bei X5 und X11, Einführen eines Teilvolumens mittels der Pumpe, Anhalten der Pumpe und erneute Druckmessung bei den Messpunkten X6 und X12 ergibt sich ein anderes Bild.

Wie dies aus Figur 2 hervorgeht, ist der Druckanstieg zwischen X5 und X6 sowie zwischen X11 und X12 vergleichsweise gering. Dies lässt den Schluss zu, dass bei dem Punkten X5 und X11 keine vollständige Entleerung des Bauchraums vorlag, was wie aus Figur 1 ersichtlich zutrifft, da zwischen den Punkten X4 und X5 sowie zwischen den Punkten X10 und X11 nur ein Teil der gesamten im Bauchraum befindlichen Dialyselösung abgelassen wurde. Der bei X5 und X11 gemessene Unterdruck (in dem Ausführungsbeispiel ca. 20 mbar) ist somit nicht repräsentativ für den peritonealen Druck.

## Patentansprüche

1. Vorrichtung zur Bestimmung des peritonealen Druckes im Bauchraum eines Patienten und/oder zur Bestimmung des Entleerungszustandes des Bauchraums eines Patienten, wobei die Vorrichtung zumindest eine Pumpe zur Förderung einer Dialyselösung in den Bauchraum sowie zumindest eine Messeinrichtung zum Messen des Druckes in der Dialyselösung umfasst, wobei die Vorrichtung wenigstens eine Steuereinheit umfasst, die derart ausgebildet ist, dass sie die Pumpe sowie die Messeinrichtung derart ansteuert, dass zum Erhalten eines ersten Druckmesswertes eine erste Messung des Druckes durch die Messeinrichtung bei stehender Pumpe vorgenommen wird, im Anschluss daran die Pumpe zur Förderung eines Teilvolumens des Gesamteinlaufvolumens der Dialyselösung in den Bauchraum in Betrieb setzt und die Pumpe dann anhält und sodann zum Erhalten eines zweiten Druckmesswertes eine zweite Messung des Druckes durch die Messeinrichtung vorgenommen wird, **dadurch gekennzeichnet, dass** die Vorrichtung zumindest eine Auswerteeinheit umfasst, die derart ausgebildet ist, dass sie die Differenz zwischen beiden Druckmesswerten ermittelt und aus der ermittelten Differenz den Entleerungszustand des Bauchraums und/oder den peritonealen Druck bestimmt, wobei die Auswerteeinheit derart ausgebildet ist, dass auf einen entleerten Bauchraum geschlossen wird, wenn die Differenz zwischen den beiden Druckwerten einen bestimmten absoluten oder relativen Grenzwert übersteigt.

2. Vorrichtung nach Anspruch 1, **dadurch gekennzeichnet, dass** die Steuereinheit derart ausgebildet ist, dass das durch die Pumpe geförderte Teilvolumen einen bestimmten Prozentsatz des gesamten, dem Patienten zu verabreichenden Gesamteinlaufvolumens nicht überschreitet oder genau einem solchen Prozentsatz entspricht.

3. Vorrichtung nach Anspruch 1, **dadurch gekennzeichnet, dass** die Steuereinheit derart ausgebildet ist, dass das durch die Pumpe geförderte Teilvolumen einem bestimmten Absolutwert entspricht oder diesen nicht überschreitet.

4. Vorrichtung nach einem der vorhergehenden Ansprüche, **dadurch gekennzeichnet, dass** die Auswerteeinheit derart ausgebildet ist, dass der peritoneale Druck dem gemessenen ersten Druckwert gleichgesetzt wird, wenn die Differenz zwischen den beiden Druckwerten einen bestimmten absoluten oder relativen Grenzwert übersteigt.

5. Vorrichtung nach einem der vorhergehenden Ansprüche, **dadurch gekennzeichnet, dass** die Vorrichtung wenigstens einen Speicher aufweist, in dem der peritoneale Druck gespeichert wird.

6. Peritonealdialysegerät umfassend wenigstens eine Vorrichtung gemäß einem der Ansprüche 1 bis 5.

7. Peritonealdialysegerät nach Anspruch 6, **dadurch gekennzeichnet, dass** das Gerät Mittel zur Erzeugung eines Alarms in Abhängigkeit des gemessenen Druckes aufweist und dass die Mittel derart ausgebildet sind, dass der ermittelte peritoneale Druck bei der Erzeugung des Alarms berücksichtigt wird.

## Claims

1. An apparatus for determining the peritoneal pressure in the abdomen of a patient and/or for determining the drainage state of the abdomen of a patient, wherein the apparatus comprises at least one pump for conveying a dialysis solution into the abdomen as well as at least one measurement device for measuring the pressure in the dialysis solution, wherein the apparatus comprises at least one control unit that is configured such that it controls the pump and the measurement device such that a first measurement of the pressure is carried out by the measurement device with a stationary pump to obtain a first measured pressure value, subsequently puts the pump into operation to convey a partial volume of the total inflow volume of the dialysis solution into the abdomen, and then stops the pump, and then a second measurement of the pressure is carried out by the measurement device to obtain a second measured pressure value, **characterized in that** the apparatus comprises at least one evaluation unit that is configured such that it determines the difference between both measured pressure values and determines the drainage state of the abdomen and/or the peritoneal pressure from the determined difference, with the evaluation unit being configured such that a conclusion is drawn on a drained abdomen when the difference between the two pressure values exceeds a specific absolute or relative limit value.

2. An apparatus in accordance with claim 1, **characterized in that** the control unit is configured such that the partial volume conveyed by the pump does not exceed a specific percentage of the overall total inflow volume to be administered to the patient or exactly corresponds to such a percentage.

3. An apparatus in accordance with claim 1, **characterized in that** the control unit is configured such that the partial volume conveyed by the pump corresponds to or does not exceed a specific absolute value.

4. An apparatus in accordance with one of the preceding claims, **characterized in that** the evaluation unit is configured such that the peritoneal pressure is considered equivalent to the first measured pressure value when the difference between the two pressure values exceeds a specific absolute or relative limit value.

5. An apparatus in accordance with one of the preceding claims, **characterized in that** the apparatus has at least one store in which the peritoneal pressure is stored.

6. A peritoneal dialysis machine comprising at least one apparatus in accordance with one of the claims 1 to 5.

7. A peritoneal dialysis machine in accordance with claim 6, **characterized in that** the machine has means for generating an alarm in dependence on the measured pressure; and **in that** the means are configured such that the determined peritoneal pressure is taken into account on the generation of the alarm.

## Revendications

1. Dispositif pour déterminer la pression intrapéritonéale dans la cavité abdominale d'un patient et/ou pour déterminer l'état de drainage de la cavité abdominale d'un patient, le dispositif comprenant au moins une pompe pour transporter une solution de dialyse dans la cavité abdominale ainsi qu'au moins un dispositif de mesure pour mesurer la pression dans la solution de dialyse, le dispositif comprenant au moins une unité de commande, qui est conçue de manière à commander la pompe ainsi que le dispositif de mesure de telle façon qu'une première mesure de la pression est effectuée par le dispositif de mesure lorsque la pompe est à l'arrêt pour obtenir une première valeur de mesure de la pression, puis à mettre en marche la pompe pour transporter un volume partiel du volume d'admission total de la solution de dialyse dans la cavité abdominale et à arrêter ensuite la pompe, une seconde mesure de la pression étant ensuite effectuée par le dispositif de mesure pour obtenir une seconde valeur de mesure de la pression, **caractérisé en ce que** le dispositif comprend au moins une unité d'évaluation, qui est conçue de manière à déterminer la différence entre les deux valeurs de mesure de la pression et déterminer l'état de drainage de la cavité abdominale et/ou la pression intrapéritonéale à partir de la différence déterminée, l'unité d'évaluation étant conçue de manière à conclure que la cavité abdominale est drainée quand la différence entre les deux valeurs de pression dépasse une valeur limite absolue ou relative déterminée.

2. Dispositif selon la revendication 1, **caractérisé en ce que** l'unité de commande est conçue de telle manière que le volume partiel transporté par la pompe ne dépasse pas un pourcentage déterminé du volume d'admission total à administrer au patient ou correspond exactement à un pourcentage de ce type.

3. Dispositif selon la revendication 1, **caractérisé en ce que** l'unité de commande est conçue de telle manière que le volume partiel transporté par la pompe correspond à une valeur absolue déterminée ou ne dépasse pas celle-ci.

4. Dispositif selon l'une des revendications précédentes, **caractérisé en ce que** l'unité d'évaluation est conçue de telle manière que la pression intrapéritonéale est assimilée à la première valeur de pression mesurée quand la différence entre les deux valeurs de pression dépasse une valeur limite absolue ou relative déterminée.

5. Dispositif selon l'une des revendications précédentes, **caractérisé en ce que** le dispositif comporte au moins une mémoire, dans laquelle la pression intrapéritonéale est enregistrée.

6. Appareil de dialyse péritonéale comprenant au moins un dispositif selon l'une des revendications 1 à 5.

7. Appareil de dialyse péritonéale selon la revendication 6, **caractérisé en ce que** l'appareil comporte des moyens destinés à générer une alarme en fonction de la pression mesurée et **en ce que** les moyens sont conçus de telle manière que la pression intrapéritonéale déterminée est prise en compte lors de la génération de l'alarme.
